# EUROPÄISCHE PATENTSCHRIFT

(11) **EP 1 149 171 B1**
(45) Veröffentlichungstag und Bekanntmachung des Hinweises auf die Patenterteilung: **22.03.2006**
(21) Anmeldenummer: 99964447.9
(22) Anmeldetag: 17.12.1999
(51) Int. Cl.: C12N 15/867, C12N 5/10, A01K 67/027, A61K 48/00

(54) **FOAMY-VIRUS-VEKTOREN ZUR EXPRESSION VON FREMDGENEN IN SÄUGERN UND DEREN VERWENDUNG**
FOAMY VIRUS VECTORS FOR EXPRESSING FOREIGN GENES IN MAMMALS AND THE USE THEREOF
VECTEURS FV POUR L'EXPRESSION DE GENES ETRANGERS CHEZ DES MAMMIFERES ET UTILISATION DESDITS VECTEURS

(30) Priorität: 17.12.1998 DE 19858441
(43) Veröffentlichungstag der Anmeldung: 31.10.2001
(73) Patentinhaber: Deutsches Krebsforschungszentrum Stiftung des öffentlichen Rechts, 69120 Heidelberg (DE)
(72) Erfinder: FLÜGEL, Rolf-M., D-69198 Schriesheim (DE); LÖCHELT, Martin, D-69207 Sandhausen (DE); FLOWER, Robert, Gore Hill, NSW 2065 (AU); WINKLER, Ingrid, Walkerville, S.A. 5081 (AU)
(74) Vertreter: Schüssler, Andrea
(86) Internationale Anmeldenummer: PCT/DE1999/004052
(87) Internationale Veröffentlichungsnummer: WO 2000/036130

(56) Entgegenhaltungen:
- WO-A-98/35024
- DE-A- 4 318 387
- HELPS CR UND HARBOUR DA: "Comparison of the complete sequence of feline spumavirus with those of the primate spumaviruses reveals a shorter gag gene" JOURNAL OF GENERAL VIROLOGY, Bd. 78, Nr. 10, Oktober 1997 (1997-10), Seiten 2549-2564, XP002135716 READING GB
- WINKLER I ET AL: "Characterization of the genome of feline foamy virus and its proteins shows distinct features different from those of primate spumaviruses" JOURNAL OF VIROLOGY, Bd. 71, Nr. 9, September 1997 (1997-09), Seiten 6727-6741, XP002135717 AMERICAN SOCIETY FOR MICROBIOLOGY US in der Anmeldung erwähnt
- SCHMIDT M ET AL: "REPLICATING FOAMY VIRUS-BASED VECTORS DIRECTING HIGH LEVEL EXPRESSION OF FOREIGN GENES" VIROLOGY,US,RAVEN PRESS, NEW YORK, NY, Bd. 210, 20. Juni 1995 (1995-06-20), Seiten 167-178, XP000674745 ISSN: 0042-6822

## Beschreibung

Die vorliegende Erfindung betrifft retrovirale, auf Foamy Virus (FV) /Spumavirus basierende Vektoren zur Einführung einer gewünschten, exprimierbaren DNA in Säugerzellen und Säugetiere, wobei der retrovirale Vektor folgende Sequenzen umfaßt: Eine erste DNA-Sequenz, die dem reversen Transkript eines Teils eines felinen FV (FeFV) entspricht, und eine zweite DNA-Sequenz, die die Propagation in Bakterien erlaubt. Die vorliegende Erfindung betrifft auch diese Vektoren enthaltende Arzneimittel.

Retroviren sind RNA-Viren, bei denen die viralen Gene von einem einzelsträngigen RNA-Molekül codiert werden. Nach Eintritt in die Wirtszelle wird die virale RNA über reverse Transkription in ein doppelsträngiges DNA-Molekül umgewandelt, im Anschluß daran gelangt diese DNA in den Nucleus und integriert sich in das Wirtsgenom als sogenanntes Provirus, das wiederum die Matrize für die Expression viraler Gene und die Synthese von Virion-RNA ist. Die viralen Genprodukte und die Virion-RNA lagern sich zu einem intakten Virion zusammen, das dann die Zelle wieder verlassen und neue Zellen infizieren kann. In der Vergangenheit konnte gezeigt werden, daß es mittels Retroviren möglich ist, Fremd-DNA in einen gewünschten Organismus einzuschleusen und dort auch zur Expression zu bringen. Somit bieten sich Retroviren grundsätzlich als Vehikel für eine Gentherapie an. Allerdings waren die damit bisher erzielten Erfolge noch nicht zufriedenstellend, da in der Regel die bei der Behandlung von Tieren bzw. Menschen verwendeten retroviralen Vektoren nur eine geringe Effizienz aufweisen. Besonders die qualitativ ungenügende und zeitlich limitierte Expression des therapeutischen Gens stellen wesentliche Limitierungen der derzeitigen retroviralen Vektoren dar (Anderson 1998, Nature 392, 25).

Somit liegt der Erfindung im wesentlichen das technische Problem zugrunde, einen viralen Vektor bereitzustellen, mit dem effizient heterologe Gene in einen gewünschten Säuger, beispielsweise Katze oder Mensch, eingeführt und effizient exprimiert werden können.

Die Lösung dieses technischen Problems wurde durch die Bereitstellung der in den Patentansprüchen gekennzeichneten Ausführungsformen erreicht.

Es konnte gezeigt werden, daß mittels eines auf einem felinen Foamyvirus (FeFV) basierenden Vektors gewünschte heterologe Gene effizient in einen gewünschten Säuger eingeführt werden können und dort auch exprimiert werden. Die Vorteile eines auf dem felinen FV basierenden Vektors beruhen vermutlich auf dessen Replikationsmechanismus, der sich von dem anderer Retroviren unterscheidet. Bespielsweise weisen FeFV einen internen Promotor zur Expression der regulatorischen "bel"-Gene auf, die für die Replikation des Virus erforderlich sind. Die Expression der "Pol"-Proteine erfolgt zudem über eine gespleißte RNA und nicht als Teil eines "Gag-Pol"-Fusionsproteins. Da das FeFV eine produktive und lebenslang persistierende Infektion der Katze mit permanenter Antigenexpression induziert, verfügt das FeFV intrinsisch über Mechanismen, die oben genannten Mängel bekannter Vektoren zu überkommen. Weiterhin sind Spumaviren physikalisch relativ stabil (minimale Titerreduktion bei Lagerung bei 4°C), sie weisen eine geringe genetische Variation auf, haben eine hohe Insertionskapazität und gelten als apathogen; Charakteristika, die die Anwendung als retrovirale Vektoren begünstigen.

Zur Herstellung eines beispielhaften Vektors wurde das gesamte Genom von felinem FV (FeFV) von den Nukleotidpositionen 17 bis zum 3' LTR in den prokaryontischen Vektor pAT153 in sequentiellen Schritten zwischen die EagI-(5'LTR) und die ClaI-(3' LTR) Restriktionsschnittstellen eingefügt. Am 5'-Ende des viralen DNA-Inserts befinden sich dabei verschiedene eingefügte Restriktionsschnittstellen. Diese rekombinante DNA ist z.B. in E. coli K12 genetisch stabil. Die biologische Aktivität der rekombinanten FeFV-DNA (Expression infektiöser Virionen) wurde nach Transfektion in permissive Zellen (CRFK) überprüft und es zeigte sich, daß infektiöse Virionen gebildet wurden. Diese unterschieden sich in ihrer Genexpression nicht von dem Ausgangsisolat (nicht-kloniertes FeFV). Darüber hinaus konnte gezeigt werden, daß bei Verwendung dieses viralen Vektors, bei dem DNA-Sequenzen, die für Neutralisationsrelevante Epitope des Env-Oberflächenproteins eines serologisch distinkten, genetisch verschiedenen FeFV-Isolats insertiert wurden, diese hererologen Domänen in transfizierten Zellen in funktionell aktiver Weise exprimiert wurden.

Somit betrifft eine Ausführungsform der vorliegenden Erfindung einen retroviralen Vektor zur Einführung einer gewünschten, exprimierbaren DNA in eine Säugerzelle, wobei der retrovirale Vektor folgende Sequenzen umfaßt: Eine erste DNA-Sequenz, die dem reversen Transkript mindestens eines Teils eines felinen FV-Genoms entspricht, und eine zweite DNA-Sequenz, die die Propagation in Bakterien und somit die effiziente und gerichtete genetische Modifikation erlaubt.

Ein weiterer Bestandteil des Vektors ist ggf. eine gewünschte, exprimierbare Fremd-DNA/therapeutisches Gen.

Der hier verwendete Ausdruck "...reverses Transkript mindestens einem Teil eines FV-Genoms entsprechend" betrifft jede FV-DNA, die noch in der Lage ist, alle Funktionen des FV-Vektors, die für die Insertion der Fremd-DNA und deren Expression nötig sind, zu gewährleisten.

Der hier verwendete Ausdruck "Sequenz, die die Propagation in Bakterien erlaubt", betrifft Sequenzen, die einen "ori" für die Replikation in Bakterien, ein Markergen und/oder Resistenzgen enthalten. Beispielsweise kann eine solche Sequenz aus pAT153 (Twiggs und Sherrat, 1980, Nature 283, S. 216) stammen.

Von den Erfindern wurde herausgefunden, daß zwei Arten von genetischer Modifikation bzw. Vektortypen vorteilhaft sind:
1) selbst-replizierende, replikationskompetente Vektoren, bei denen die Insertion heterologer Gene die Replikationsfähigkeit nicht beeinträchtigen und die im therapeutischen Wirt replizieren können. Bei den selbstreplizierenden Vektoren ist keine Packaging-Zellinie oder ein Helfervirus notwendig;
2) replikationsdefekte Vektoren, bei denen z.B. die gesamten Strukturgene (gag, env, pol) durch die Fremd-DNA ersetzt wurden. Die Virionenbildung erfolgt in sog. Packaging-Zellinien, die alle deletierten Funktionen in trans zur Verfügung stellen (gag, env, pol). In diesen Vektoten sind die für die Verpackung der Genome und Expression der Fremd-DNA notwendigen cis-Sequenzen erhalten.

Spumaretroviren/Foamyviren gelten allgemein als apathogen, obschon sie unter bestimmten experimentellen (artifiziellen) Bedingungen (transgene Mäuse) Krankheiten auslösen können. Die "apparente Apathogenität" von Foamyviren ist einer ihrer großen Vorteile als retroviraler Vektor. Bei allen selbstreplizierenden FeFV-Vektoren sollte jedoch bevorzugt der starke Bel 1-Transaktivator zumindest funktionell inaktiviert, vorzugsweise aber komplett deletiert sein.

Die in die erfindungsgemäßen Vektoren einbringbare Fremd-DNA unterliegt keiner Beschränkung und es sind prinzipiell alle Krankheits-relevanten Gene möglich. Beispiele für Fremd-DNA (auch heterologe Gene oder therapeutische Gene genannt) sind
- Immunmodulatorische Gene zur Aktivierung oder Suppression von Immunreaktionen im Zuge von Anti-Krebstherapien, z.B. Interleukin-2, -1, -10 etc., gamma-Interferon, Tumor Nekrosis Faktor, spezielle Tumorantigene
- Suizidgene zur spezifischen Destruktion der entsprechend infizierten Zellen, z.B. HSV Thymidin-Kinase, E. coli Cytosin Deaminase, Polynukleosid-Phosphorylase
- funktionell intakte Gene zur Substitution funktionell defekter Gene im Zuge der somatischen Gentherapie,
- Expression von trans-dominant negativen Mutanten-Proteinen, die inhibitorisch im Rahmen der "pathogen derived resistance" oder "intrazellulären Immunisierung" eingesetzt werden,
- Gene, deren Proteinprodukt aus transgenen Tieren in geeigneter Form in technisch/indutriellem Maßstab insoliert und gereinigt werden kann, z.B. Faktor X der Blutgerinnung oder dgl.

Erfindungsgemäß handelt es sich bei dem retroviralen FV-Vektor um einen Vektor bei dem das Genom des felinen Foamyvirus (FeFV) verwendet wurde. Dabei kann es sich um einen Vektor handeln, bei dem die erste DNA-Sequenz die FV-DNA-Sequenz des Vektors FeFV-7 ist. Dieser Vektor wurde als Plasmid pFeFV-7 bei der DSMZ, Mascheroder Weg 1b, 38124 Braunschweig am 23. November 1998 unter DSM 12514 hinterlegt.

Die erste DNA-Sequenz des Vektors kann sich jedoch von der DNA-Sequenz des Vektors pFeFV-7 unterscheiden und lediglich von ihr abgeleitet sein. Der FeFV-Anteil des Plasmids kann beispielsweise in der Weise modifiziert sein, daß er entsprechend den Anforderungen des Vektorkonzepts (selbstreplizierender oder replikationsdefekter Vektor) genetisch verändert wird und die Fremd-DNA (oben auch als heterologes Gen bzw. therapeutisches Gen bezeichnet) unter der transkriptionellen Kontrolle des FeFV oder eines heterologen Promotors insertiert wird. Beispielsweise kann sich die erste DNA-Sequenz auch von der des erfindungsgemäßen Vektors pFeFV-7 bezüglich der Länge unterscheiden, oder verschiedene Nukleotidadditionen, -deletionen oder substitutionen aufweisen, beispielsweise von einem anderen Feldisolat von FeFV stammen, solange die gewünschten Eigenschaften des Virus, beispielsweise hinsichtlich Infektiosität, Replikation, Insertion und Expression der Fremd-DNA für die gewünschten erfindungsgemäßen Zwecke erhalten bleiben. Der Fachmann kann mittels üblicher Methoden, beispielsweise den in den nachfolgenden Beispielen oder in Winkler et al., J.Virol.71 (1997), 6727-6741, angegebenen Verfahren bestimmen, ob die für die Herstellung des retroviralen FeFV-Vektors verwendete DNA-Sequenz noch die erforderlichen Bedingungen erfüllt. Darüber hinaus können allgemeine, auf dem Fachgebiet bekannte Verfahren zur Konstruktion der erfindungsgemäßen retroviralen Vektoren verwendet werden. Zu diesen Verfahren zählen beispielsweise übliche in vitro-Rekombinationstechniken, synthetische Verfahren, sowie in vivo-Rekombinationsverfahren.

Die gewünschte, zu exprimierende Fremd-DNA kann an jeder Stelle des FV-Genoms inseriert werden, vorausgesetzt, daß dadurch deren Expression gewährleistet ist, sowie die gewünschten Eigenschaften des FV (beispielsweise selbtsreplizierender oder replikationsdefekter Vektor) erhalten bleiben. Prinzipiell kann die Fremd-DNA an jeder Stelle des FeFV-Genoms insertiert werden. Es sollte allerdings so sein, daß der FeFV-Anteil immer noch in dem oben angesprochenen Sinne (selbst-replizierender/replikations-defekter Vektor) aktiv ist. Vorzugsweise wird die Fremd-DNA-Sequenz zwischen den 5'LTR-Bereich und den 3'LTR-Bereich inseriert. Bei selbstreplizierenden Vektoren können heterologe Sequenzen in die Gag, Env und Be12-Gene bzw. in definierte Teile der 3'-LTR eingefügt werden, bei replikations-defekten Vektoren werden bevorzugt die Gag, Pol, Env und akzessorischen/regulatorischen Gene durch die zu exprimierende Fremd-DNA ersetzt. Um eine effiziente Expression der Fremd-DNA-Sequenz zu erhalten, ist es erforderlich, diese so in den FV-Vektor zu inserieren, daß sie mit geeigneten transkriptionellen Kontrollsequenzen funktionell verknüpft vorliegt. Geeignete, für die Expression in Säugern geeignete Kontrollsequenzen (d.h. Promotor- und/oder Enhancer-Sequenzen) sind dem Fachmann bekannt. Als Promotoren sind die dem Fachmann bekannten Promotoren verwendbar. Diese umfassen beispielsweise
- den genetisch modifizierten FeFV LTR-Promotor oder den internen Promotor
- konstitutiv aktive Promotoren, z.B. HSV-tk, CMV-IE
- zelluläre housekeeping Promotoren, z.B. beta-Actin, GADPH
- zelltyp-spezifische Promotoren für die Expression in haematopoetischen Zellen, dem ZNS, der Leber, Niere etc.
- induzierbare Promotoren, z.B. Insulin-, Corticoid-, Stress-, Östrogen-abhängige Promotoren
- virus-aktivierbare Promotoren, z.B. HIV LTR für intrazelluläre Immunisierungen
- Promotoren, die durch etablierte Therapeutika aktiviere werden (Tamoxifen etc.)

In einer besonders bevorzugten Ausführungsform enthält der retrovirale FV-Vektor außerdem ein Gen, das einen nachweisbaren phänotypischen Marker codiert. Dies erlaubt die Überprüfung einer erfolgreich verlaufenen Transformation der gewünschten Zielzelle. Geeignete Markergene sind beispielsweise:
- β-Galactosidase
- Resistenzgene gegen Neomycin, Hygromycin, Zeonin etc.
- (humanisiertes) green fluorescent Protein GFP

Die vorliegende Erfindung betrifft ferner ein Plasmid, das den erfindungsgemäßen FeFV-Vektor enthält. Das Plasmid kann beispielsweise in E. coli, z.B. E. coli JM 109 oder DH5α, vermehrt werden und aus diesen direkt isoliert werden.

Verfahren zur Transformation der Zellen zur Herstellung des erfindungsgemäßen FV-Vektors (beispielsweise über CaPO₄₋Präzipitation, Liposomen oder Elektrotransformation etc.) und zur phänotypischen Selektion von Transformanten sind auf dem Fachgebiet bekannt.

Nach erfolgter Vermehrung können die erfindungsgemäßen FV-Vektoren in eine Zelle, ein Gewebe, Organ, einen Patienten oder ein Tier durch eine Reihe von Verfahren eingeführt werden. Das FeFV (und somit auch das davon abgeleitete Plasmid pFeFV-7) kann in Zellen humanen Ursprungs replizieren. Das das FeFV diese humanen Zellen auch infizieren kann, sind keine absolut essentiellen Veränderungen des Plasmids pFeFV und seiner Derivate für die Replikation und Applikation in humanen Zellen und den Menschen notwendig. Für die ex-vivo Applikation kommen die vorgenannten Verfahren sowie Co-Kultivierung mit Vektor-produzierenden Zellen und direkte Infektion mit dem retroviralen Vektor in Frage. Für die in-vivo Applikation kommen in Frage: als DNA durch die Gen-Gun; Liposomen-Technik; als freies Retrovirus systemisch (z.B. intravenös), lokal (z.B. direkt in einen Tumor oder das lymphatische Organ) oder in Form eines Aerosols für den Respirationstrakt. Es können analog auch die Vektor-produzierenden Zellen verabreicht werden.

Somit betrifft die vorliegende Erfindung auch Zellen und transgene nicht-menschliche Tiere (d.h. Säuger, die bezüglich der mittels des erfindungsgemäßen FV-Vektors eingeschleusten DNA-Sequenz transgen sind). Verfahren zur Herstellung solcher transgener Tiere können beispielsweise in WO 91/08216 oder Schenkel, Johannes, 1995, Transgene Tiere, Spektrum Akad. Verlag, Heidelberg gefunden werden.

Die vorliegende Erfindung betrifft ferner Arzneimittel, die die vorstehend beschriebenen FV-Vektoren enthalten. Diese Arzneimittel enthalten gegebenenfalls zusätzlich einen pharmazeutisch verträglichen Träger. Geeignete Träger und die Formulierung derartiger Arzneimittel sind dem Fachmann bekannt. Zu geeigneten Trägern zählen beispielsweise Phosphatgepufferte Kochsalzlösungen, Wasser, Emulsionen, beispielsweise Öl/Wasser-Emulsionen, Netzmittel, sterile Lösungen etc. Die Art des Trägers hängt natürlich davon ab, wie der erfindungsgemäße FV-Vektor verabreicht werden soll. Die geeignete Dosierung wird von dem behandelnden Arzt bestimmt und hängt von verschiedenen Faktoren ab, beispielsweise von dem Alter, dem Geschlecht, dem Gewicht des Tiers bzw. Patienten, dem Schweregrad der Erkrankung, der Art der Verabreichung etc..

Der erfindungsgemäße FV-Vektor kann beispielsweise auch für die Expression von DNA-Sequenzen, die für neutralisierende Epitope pathogener Erreger codieren, verwendet werden, d.h. er kann für eine Vakzinierung gegenüber diesen Erregern verwendet werden. Dies ist vor allem hinsichtlich solchen infektiösen Erkrankungen, beispielsweise bei Katzen, interessant, für die bisher keine geeigneten Impfstoffe zur Verfügung standen. In diesem Zusammenhang ist zu beachten, daß es sich bei den exprimierten Epitopen um therapeutische (immunologisch) wirksame Domänen handelt und die Vakzinierung nicht z.B. zu einer Immunpathogenese bei nachfolgender virus-Challenge führt. Beispielsweise kann die Fremd-DNA-Sequenz für neutralisierende Epitope des felinen Immundefizienzvirus (FIV) codieren. Dabei wird ein FV-Vektor erhalten, der die effiziente Vakzinierung von Katzen gegenüber FIV erlaubt. Andere geeignete Fremd-DNA sind virale Env-Oberflächendomänen oder T-Zellepitope von viralen Struktur- oder Nichtstrukturproteinen. Wie bereits oben ausgeführt ist die Verwendung der erfindungsgemäßen Gegenstände aber keineswegs auf die Vakzinierung beschränkt, sondern stellt allgemein ein Vehikel für die Gentherapie dar.

Um die Praktikabilität von FeFV-basierten Vektoren zu demonstrieren, wurden die für die Virus-Neutralisation relevanten Epitope des von den Erfindern verwendeten Isolats FUV durch die entsprechenden Sequenzen eines Isolats 951-ähnlichen FeFV-Virus (Flower et al., 1985, Arch. Virol. 83, S. 53) ausgetauscht. Das genetisch modifizierte Virus ist replikationskompetent und wird aufgrund des Austausches von Neutralisations-relevanten Epitopen nicht mehr durch Seren gegen das FeFV-FUV-Isolat neutralisiert werden. Dieses genetisch modifizierte, nun 951-ähnliche FeFV kann eventuell aufgrund seiner distinkten immunologischen Charaktieristika in Katzen (oder Menschen) dann als gentherapeutischer Vektor eingesetzt werden, wenn bereits eine humorale Immunität gegen den ursprünglichen, FW-ähnlichen Vektor, vorliegt. Eine solche Situation kann vorliegen, wenn der therapeutische Einsatz des Vektors wiederholt durchgeführt werden muß. Somit kann die Verfügbarkeit der immunologisch distinkten FeFV-Vektoren einen wesentlich flexibleren Einsatz dieser rekombinaten Viren in der Therapie erlauben. Therapeutische Applikationen sind trotz bestehender Immunität gegen ein FeFV-Virus-Isolat immer noch mit dem anderen FeFV-Serotyp möglich, was ein Vorteil ist, den keines der anderen retroviralen Vektorsysteme aufweist.

Der erfindungsgemäße FV-Vektor ist jedoch nicht nur für die Gentherapie/Vakzinierung von Tieren, beispielsweise Katzen geeignet, sondern auch für entsprechende Therapien beim Menschen. Aufgrund der Tatsache, daß (a) mit dem vorstehend beschriebenen erfindungsgemäßen FeFV-Vektor Katzen gegen FIV geimpft werden können und (b) die FIV-Infektion der Katze molekularbiologisch und pathogenetisch sehr stark der HIV-Infektion des Menschen ähnelt (siehe beispielsweise dazu Elder et al., Aids Research and Human Retroviruses 14 (1998), 797-801), kann davon ausgegangen werden, daß der erfindungsgemäße FV-Vektor nicht nur zur Gentherapie beim Menschen hinsichtlich beispielsweise der Einführung von Tumorsuppressorgenen geeignet ist, sondern auch zur Durchführung einer Schutzimpfung gegenüber HIV von Nutzen ist. Für die Vakzinierung gegen die HIV-Infektion bieten sich primär T-Zell Epitope und Sequenzen der HIV Struktur- und Nichtstrukturproteine an, die einerseits immunrelevante Epitope tragen und gleichzeitig zumindest moderat konserviert sind. Von den Erfindern wurde bestätigt, daß feline und humane Zellen den erfindungsgemäßen Vektor replizieren, und rekombinante Vektorpartikel werden in beiden Zelltypen (z.B. feline CRFK-Zellen, humane 293T-Zellen) gebildet.

Schließlich betrifft somit die vorliegende Erfindung auch die Verwendung des erfindungsgemäßen FV-Vektors zur Vakzinierung, vorzugsweise gegenüber FIV bei Katzen oder HIV beim Menschen.

Die Figuren zeigen:

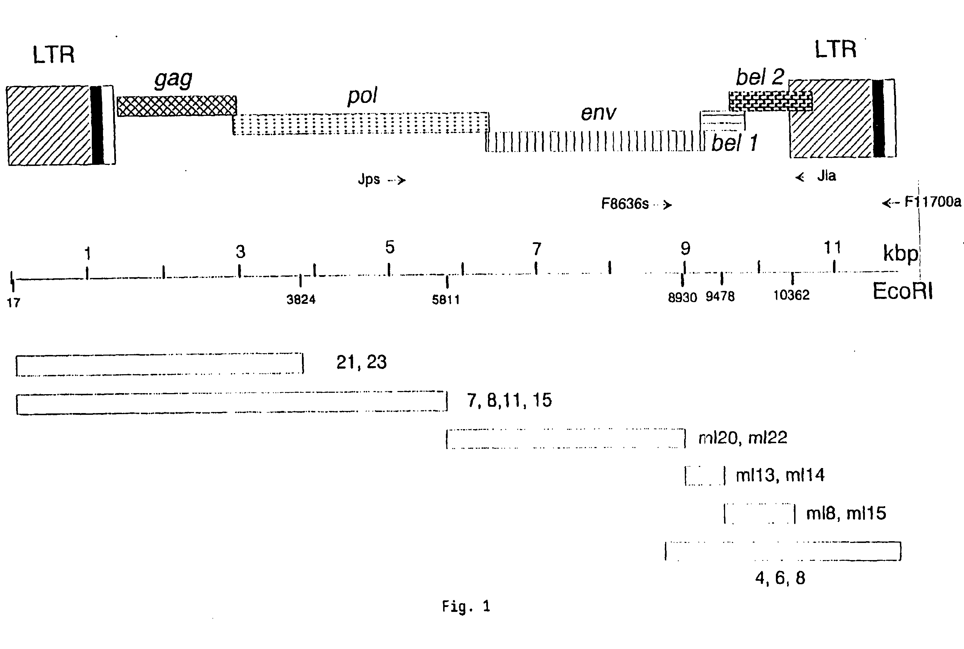

- Fig 1:: Schematische Darstellung der Organisation des FeFV- Provirus

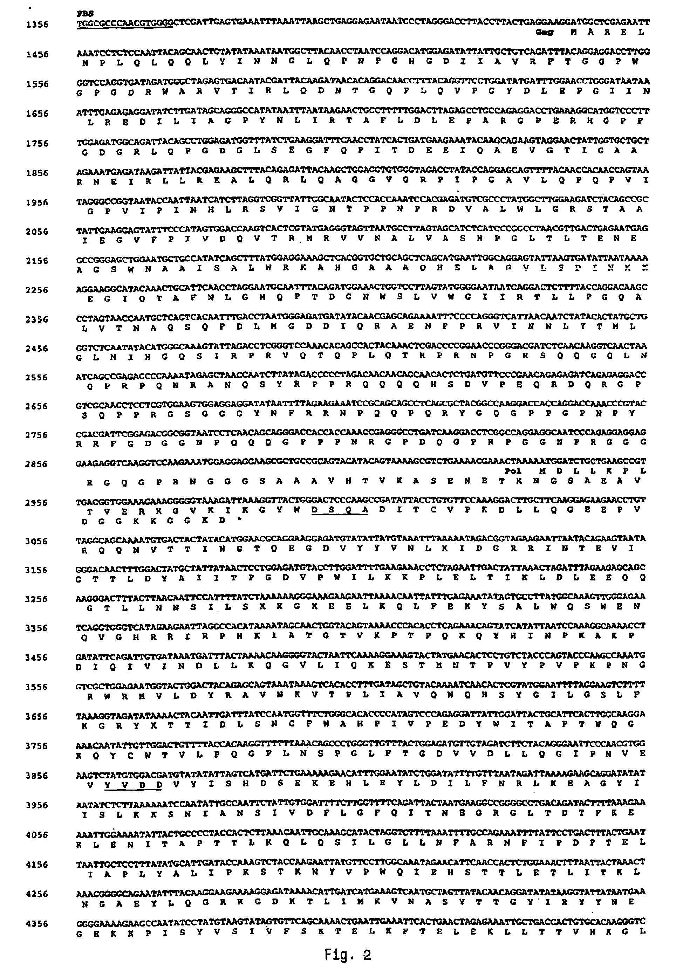

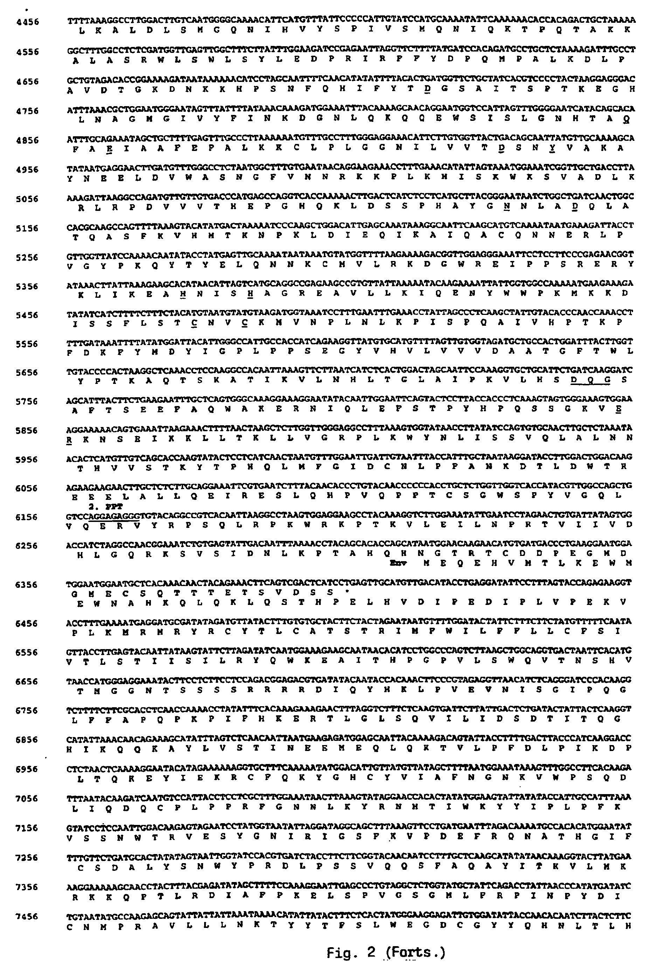

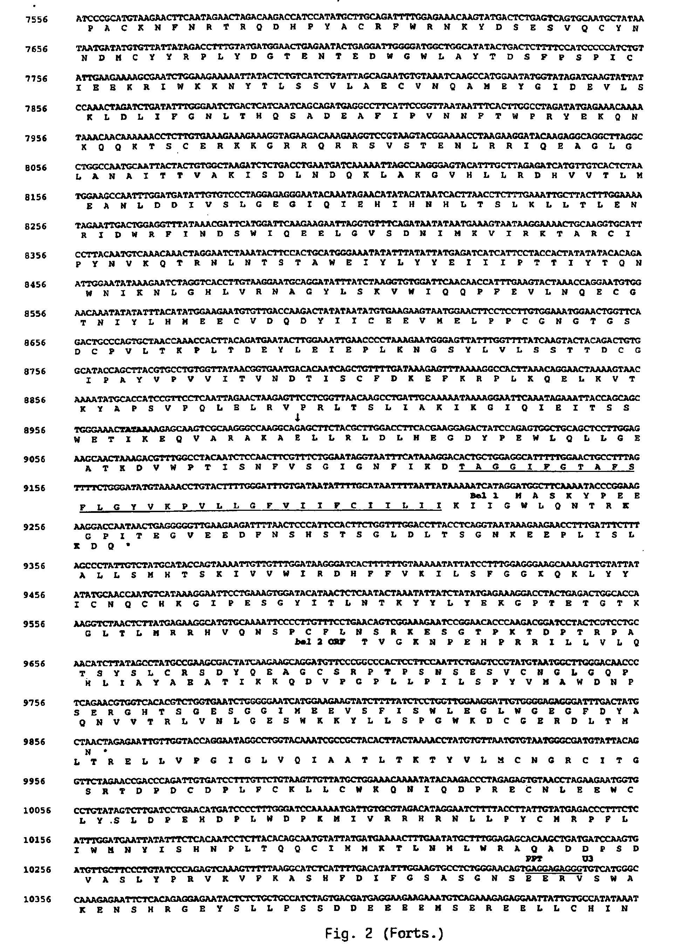

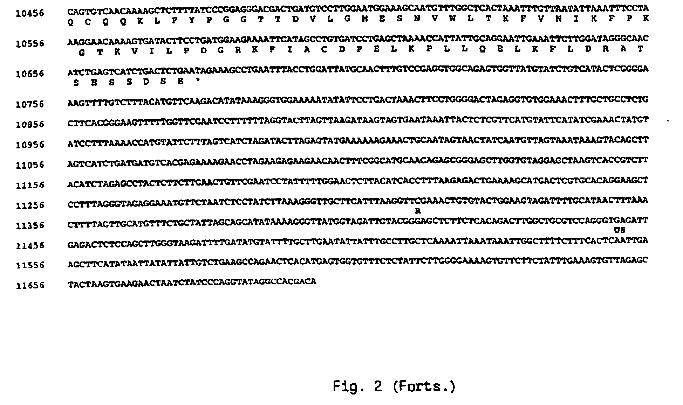

- Fig.2:: Nucleinsäure-Sequenz und abgeleitete Aminosäuresequenz von FeFV (Winkler et al., J. of Virology, Vol. 71, No. 9, Sept. 1997, S. 6727-6741)

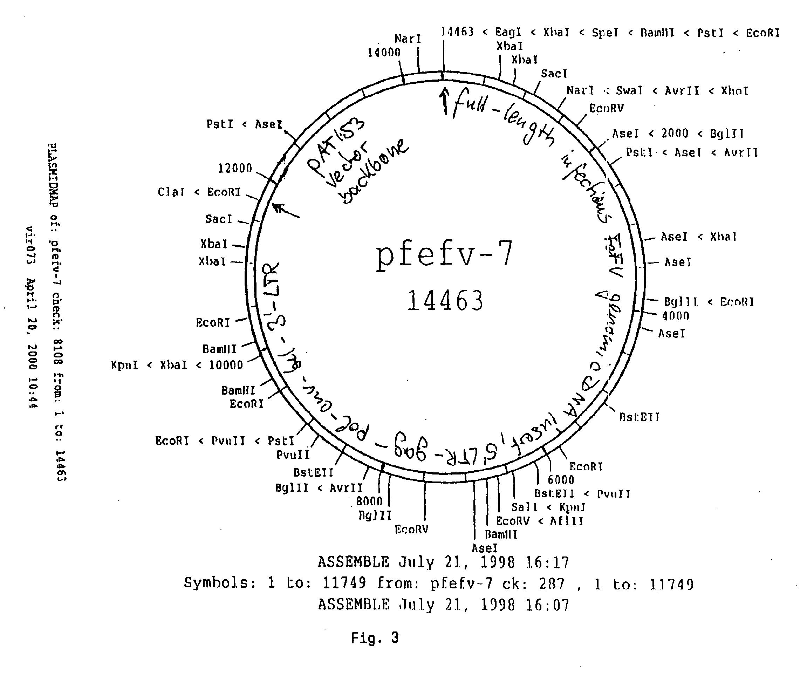

- Fig.3:: Karte des Clons pFeFV-7 (Länge: 14.463 bp)
Die pAT153 und die FeFV entsprechenden Anteile sind gekennzeichnet. Außerdem sind die Positionen der wichtigsten Restriktionsschnittstellen angegeben.

Die nachstehenden Beispiele veranschaulichen die Erfindung.

### Beispiel 1

### Herstellung eines FeFV-Vektors (pFeFV-7)

Die Gewinnung von FeFV-Isolaten, die DNA-Extraktion, die Konstruktion von rekombinanten FeFV-DNA-Clonen und die Analyse dieser Clone erfolgte im Prinzip wie in Winkler er al., J.Virol.71 (1997), 6727-6741, beschrieben. FeFV-DNA-Sequenzen von den Nucleotidpositionen 5118 bis 7431 wurden durch "long-template PCR" unter Verwendung spezifischer Primer und der DNA von mit FeFV infizierten "Crandell"-Katzennierenzellen (CRFK-Zellen) durchgeführt. Die Primer sind:

Die erhaltene amplifizierte DNA wurde in pCRII-Vektoren (Fa. Invitrogen, Niederlande) mittels üblicher Verfahren cloniert. Korrekte recombinante DNA-Clone wurden mit NdeI (in FeFV) und Ec1136II (im pCRII-Vektor) geschnitten und das erhaltene Fragment von etwa 2,3 kbp wurde in den FeFV-DNA-Clone 7 (der mit ClaI gespalten, danach mittels Klenow-Polymerase mit glatten Enden versehen und schließlich mit NdeI geschnitten worden war) inseriert. Clone 7 enthält FeFV-DNA-Sequenzen von den Nucleotidpositionen 17 bis 5811 (Winkler et al., 1997). Durch diese Clonierung wurden FeFV-Clone erhalten, die sich von der Nucleotidposition 17 bis 7431 erstreckten (Clone 24 und 28).

Gleichzeitig wurde die FeFV-Insertion aus den rekombinanten Clonen 4, 6 und 8, die FeFV-DNA-Sequenzen von den Nucleotidpositionen 8636 bis zum 3'-Ende des 3'LTR enthielten (Winkler et al., 1977)*,* in den Vektor pBluescript KS (Fa. Stratagene, Heidelberg) subcloniert, wobei die gemeinsamen flankierenden Restriktionsenzymschnittstellen des Polylinkers verwendet wurden. In den erhaltenen Clonen 5,7 und 15 schließen sich an das 3'Ende des Genoms ClaI- und ApaI-Schnittstellen von den PCR-Primern und der Polylinkerstelle des Vektors an. FeFV-DNA-Sequenzen von den Nucleotidpositionen 7163 bis 9057 wurden durch "long-template PCR" unter Verwendung der oben genannten Primer amplifiziert und in das Plasmid pCRII wie vorstehend beschrieben cloniert, wobei der Clone V erhalten wurde.

Zur Herstellung eines Clons, der die vollständige provirale DNA enthält, wurden die folgenden clonierten, vorstehend beschriebenen DNA-Fragmente in einer Dreifachligation miteinander verknüpft: Das ApaI(Vektorschnittstelle)/Pml I-Fragment der Clone 24/28 + das PmlI/PstI-Fragment der Clone V + das PstI/ApaI-Fragment der Clone 5, 7 und 15.

Die erhaltenen DNA-Clone waren genetisch stabil und enthielten FeFV-DNA von den Nucleotidpositionen 17 bis 11700 als Insertion im pBluescript-Vektor. Die FeFV-Insertion zwischen den EagI- und ClaI-Stellen, die die FeFV-DNA flankieren, wurden dann in den entsprechend geschnittenen Vektor pAT153 (= "high copy-number" Deletionsmutante von pBR322; Twiggs und Sherrat, 1980, Nature 283, S. 216) subcloniert.

Verschiedene, unabhängig erhaltene Clone wurden nach Transfektion in FeFV-permissive eukaryontische CRFK-Zellen analysiert; es zeigte sich jedoch ein geringe Infektiosität. Clone 13 zeigte eine moderate virale Infektiosität und wurde daher zur vollständigen Restaurierung der Infektiosität verwendet. FeFV-DNA-Sequenzen von den Nucleotidpositionen 5775 bis 10522 wurde mittels "long-template PCR" unter Verwendung der oben genannten Primer amplifiziert und direkt mit BstZ17I (Nucleotidposition 5980) und BsaI (Nucleotidposition 10137) geschnitten. Das erhaltene DNA-Fragment mit einer Länge von etwa 4,2 kbp wurde zum Austausch der entsprechenden Sequenzen des Clons 13 (mit vollständiger Länge) verwendet. Nach Transfektion in CRFK-Zellen zeigte sich, daß der erhaltene FeFV-Clone pFeFV-7 voll infektiös und genetisch stabil war. Das erhaltene recombinante FeFV-Virus war von nichtclonierten, aus Zellkulturen erhaltenen Viren nicht unterscheidbar. pFeFV-7 wurde am 23. November 1998 unter der Nummer DSM 12514 bei der DSMZ, Mascheroder Weg 2, Braunschweig hinterlegt.

### Beispiel 2

### Herstellung einer Neutralisations-resistenten Variante von pFeFV-7, die heterologe Sequenzen des FeFV-Serotyps 951 in Katzenzellen exprimiert

Um die Praktikabilität von FeFV-basierten Vektoren zu demonstrieren, wurden die für die Virus-Neutralisation relevanten Epitope des oben beschriebenen FeFV-7 Isolats durch die entsprechenden Sequenzen eines Isolat 951-ähnlichen FeFV-Virus (Flower et al., 1985, Arch. Virol. 83, 53) ausgetauscht. Dabei wurde wir folgt vorgegangen:
Die Env DNA-Sequenzen des FeFV-Isolats 951 wurden aus DNA von FeFV-951-infizierten CRFK-Zellen mit dem forward-Primer 5-GACATACCTGAAGATATTC-3'(Position 6418) und dem reverse-Primer 5'-CGACTTGTACCAGGCCTATTCCTGG-3'(Position 9901) mittels PCR amplifiziert. Das Amplifikat wurde mit der Restriktionsendonuklease KpnI verdaut und das ca. 3,5 kB große DNA-Fragment isoliert. Parallel wurde der oben beschriebene FeFV-Vektor pFeFV-7 mit Kpn verdaut und das dem Vektor-Backbone entsprechende DNA-Fragment isoliert. Beide DNAs wurden miteinander ligiert und die Rekombinanten identifiziert, bei denen die FeFV-951 env DNA-Sequenzen in der korrekten Orientierung in pFeFV-7 insertiert worden waren. Der resultiernden DNA-Klon pFEFV-7/951 ist genetisch stabil. Nach Transfektion in CRFK-Zellen ist das Proteinexpressionsmuster dieses Klons nicht von dem Wildtyp-Klon pFeFV-7 unterscheidbar. Plasmid pFeFV-7/951 induziert die Synthese infektiöser FeFV-Partikel.

Das genetisch modifizierte Hybrid pFeFV-7/951 ist replikationskompetent und wird aufgrund des Austauschs von Neutralisations-relevanten Epitopen nicht mehr durch Seren gegen das FeFV-Isolat neutralisiert. Dies wurde in Neutralisationsstudien gezeigt: Seren, die gegen das FEFV-Isolat gerichtet sind, neutralisieren Nachkommen des Plasmids pFeFV-7, nicht aber Viren des Klons pFEFV-7/951. Seren gegen das FeFV-Isolat 951 neutralisieren entsprechend Viren des Klons pFeFV-7/951, nicht jedoch vom Plasmid pFeFV-7.

Es wurde in diesem Beispiel somit ein chimärer Hybridvektor konstruiert, der heterologe Protein-Sequenzen effizient und stabil exprimiert. Zudem wurde erstmals ein vektorpaar (Plasmids pFeFV-7 ind pFeFV-7/951) konstruiert, das keine kreuzneutralisierenden Antikörper induziert.

In einer therapeutischen Anwendung beider Vektoren kann also zuerst ein Serotyp (z.B. FeFV-7) für den Transfer des therapeutischen Gens eingesetzt werden. Etabliert der Patient eine neutralisierende Seroreaktivität gegen diesen Vektor-Serotyp, kann der andere Serotyp (in diesem Fall pFeFV-7/951) eingesetzt werden, gegen den keine neutralisierenden Antikörper vorliegen. Therapeutische Applikationen wären trotz bestehender Immunität gegen ein Virus-Isolat immer noch mit dem anderen Serotyp möglich. Dies ist ein Vorteil, den kein anderes retrovirales Vektorsystem aufweist.

## Patentansprüche

1. Retroviraler Vektor zur Einführung einer gewünschten, exprimierbaren DNA in eine Säugerzelle, wobei der retrovirale Vektor folgende Sequenzen umfaßt: Eine erste DNA-Sequenz, die dem reversen Transkript mindestens eines Teils eines felinen Foamyvirus (FeFV) entspricht, und eine zweite DNA-Sequenz, die die Propagation in Bakterien erlaubt.

2. Retroviraler Vektor nach Anspruch 1, wobei in dem Vektor weiter eine gewünschte exprimierbare Fremd-DNA enthalten ist.

3. Retroviraler Vektor nach Anspruch 1 oder 2, wobei die erste DNA-Sequenz das reverse Transkript des gesamten Foamyvirus umfaßt.

4. Retroviraler Vektor nach einem der Ansprüche 1 bis 3, wobei die erste DNA-Sequenz sowohl die 5' LTR als auch die 3'LTR eines Foamyvirus umfaßt.

5. Retroviraler Vektor nach einem der Ansprüche 1 bis 4, wobei die zweite DNA-Sequenz in den 5'LTR-Bereich und/oder 3'LTR-Bereich des Foamyvirus inseriert ist.

6. Retroviraler Vektor nach einem der Ansprüche 1 bis 5, der zusätzlich ein Gen enthält, das einen nachweisbaren phänotypischen Marker codiert.

7. Retroviraler Vektor nach einem der Ansprüche 2 bis 6, wobei die Fremd-DNA für neutralisierende Epitope des felinen Immundefizienzvirus (FIV) oder des menschlichen Immundefizienzvirus (HIV) codiert.

8. Retroviraler Vektor nach einem der Ansprüche 1 bis 7, wobei die erste DNA-Sequenz jene des bei der DSMZ am 23. November 1998 hinterlegten Plasmids pFeFV-7 (DSM 12514) ist.

9. Plasmid, den retroviralen Vektor nach einem der Ansprüche 1 bis 8 enthaltend.

10. Zelle, das Plasmid nach Anspruch 9 enthaltend.

11. Zelle nach Anspruch 10, die eine Tierzelle ist.

12. Zelle nach Anspruch 11, die eine Säugerzelle ist.

13. Transgenes, nicht-menschliches Tier, den retroviralen Vektor nach einem der Ansprüche 1 bis 8 oder das Plasmid nach Anspruch 9 enthaltend.

14. Verwendung des Vektors nach einem der Ansprüche 1 bis 8 oder des Plasmids nach Anspruch 9 zur Herstellung eines Arzneimittels zur Vakzinierung.

15. Verwendung des Vektors nach Anspruch 7 zur Herstellung eines Arzneimittels zur Vakzinierung von Katzen gegen FIV oder Menschen gegen HIV.

16. Verwendung des Vektors nach einem der Ansprüche 1 bis 8 oder des Plasmids nach Anspruch 9 zur Herstellung eines Arzneimittels zur gentherapeutischen Behandlung von Patienten.

## Claims

1. A retroviral vector for introducing a desired, expressible DNA into a mammalian cell, wherein the retroviral vector comprises the following sequences: A first DNA sequence corresponding to the reverse transcript of at least part of a feline foamy virus (FeFV), and a second DNA sequence permitting the propagation in bacteria.

2. The retroviral vector according to claim 1, wherein the vector further comprises a desired expressible foreign DNA.

3. The retroviral vector according to claim 1 or 2, wherein the first DNA sequence comprises the reverse transcript of the entire foamy virus.

4. The retroviral vector according to any one of claims 1 to 3, wherein the first DNA sequence comprises both 5'LTR and 3'LTR of a foamy virus.

5. The retroviral vector according to any one of claims 1 to 4, wherein the second DNA sequence is inserted in the 5'LTR region and/or 3'LTR region of the foamy virus.

6. The retroviral vector according to any one of claims 1 to 5, which additionally contains a gene coding for an identifiable phenotypic marker.

7. The retroviral vector according to any one of claims 1 to 6, wherein the second DNA sequence codes for neutralizing epitopes of the feline immunodeficiency virus (FIV) or the human immunodeficiency virus (HIV).

8. The retroviral vector according to any one of claims 1 to 7, wherein the first DNA sequence is that of the plasmid pFeFV-7 deposited with the DSMZ (German Type Collection of Microorganisms and Cell Cultures) on November 23, 1998 (DSM 12514).

9. A plasmid containing the retroviral vector according to any one of claims 1 to 8.

10. A cell containing the plasmid according to claim 9.

11. The cell according to claim 10, which is an animal cell.

12. The cell according to claim 11, which is a mammalian cell.

13. A transgenic non-human animal containing the retroviral vector according to any one of claims 1 to 8 or the plasmid according to claim 9.

14. Use of the vector according to any one of claims 1 to 8 or the plasmid according to claim 9 for the production of a medicament for vaccination.

15. Use of the vector according to claim 7 for the production of a medicament for the vaccination of cats against FIV or human beings against HIV.

16. Use of the vector according to any one of claims 1 to 8 or the plasmid according to claim 9 for the production of a medicament for the gene-therapeutic treatment of patients.

## Revendications

1. Vecteur rétroviral pour introduire un ADN exprimable souhaité dans une cellule de mammifère, le vecteur rétroviral comprenant les séquences suivantes : une première séquence d'ADN correspondant à la transcription inverse au moins d'une partie d'un virus Foamy félin (FeFV), et une deuxième séquence d'ADN permettant la propagation de bactéries.

2. Vecteur rétroviral selon la revendication 1, dans lequel un ADN étranger exprimable souhaité est en outre contenu dans le vecteur.

3. Vecteur rétroviral selon la revendication 1 ou 2, dans lequel la première séquence d'ADN comprend la transcription inverse du virus Foamy global.

4. Vecteur rétroviral selon l'une des revendications 1 à 3, dans lequel la première séquence d'ADN comprend aussi bien l'extrémité 5' de la LTR que l'extrémité 3' de la LTR d'un virus Foamy.

5. Vecteur rétroviral selon l'une des revendications 1 à 4, dans lequel la deuxième séquence d'ADN est insérée dans l'extrémité 5' de la région LTR et/ou l'extrémité 3' de la région LTR du virus Foamy.

6. Vecteur rétroviral selon l'une des revendications 1 à 5, qui contient en outre un gène codant un marqueur phénotypique référençable.

7. Vecteur rétroviral selon l'une des revendications 2 à 6, dans lequel l'ADN étranger code l'épitope neutralisant du virus de l'immuno-déficience féline (VIF) ou du virus de l'immuno-déficience humaine (VIH).

8. Vecteur rétroviral selon l'une des revendications 1 à 7, dans lequel la première séquence d'ADN est celle du plasmide pFeFV-7 (DSM 12514) consigné lors du SDMZ du 23 novembre 1998.

9. Plasmide, contenant le vecteur rétroviral selon l'une des revendications 1 à 8.

10. Cellule, contenant le plasmide selon la revendication 9.

11. Cellule selon la revendication 10, qui est une cellule animale.

12. Cellule selon la revendication 11, qui est une cellule de mammifère.

13. Transgènes, d'animal non humain, contenant le vecteur rétroviral selon l'une des revendications 1 à 8 ou le plasmide selon la revendication 9.

14. Utilisation du vecteur selon l'une des revendications 1 à 8 ou du plasmide selon la revendication 9 pour fabriquer un remède pour la vaccination.

15. Utilisation du vecteur selon la revendication 7 pour fabriquer un médicament pour la vaccination des félins contre le VIF ou des humains contre le HIV.

16. Utilisation du vecteur selon l'une des revendications 1 à 8 ou du plasmide selon la revendication 9 pour fabriquer un médicament pour le traitement de patients par thérapie génique.


### SEQUENZ PROTOKOLL

<110> Deutsches Krebsforschungszentrum
<120> FV-Vektoren zur Expression von Fremdgenen in Säugern und deren Verwendung
<130> K 2769
<140> PCT/DE99/04052
   <141> 1999/12/17
<150> DE 198 58 441.5
   <151> 1998/12/17
<160> 6
<170> PatentIN Ver. 2.1
<210> 1
   <211> 28
   <212> DNA
   <213> Künstliche Sequenz
<220>
   <223> Primer
<400> 1
   CCTCATGCTT ACGGGAATAA TCTGGCTG 28
<210> 2
   <211> 28
   <212> DNA
   <213> Künstliche Sequenz
<220>
   <223> Primer
<400> 2
   GAATAGCATA CCAGAGCCTA CAGGGCTC 28
<210> 3
   <211> 28
   <212> DNA
   <213> Künstliche Sequenz
<220>
   <223> Primer
<400> 3
   CCAATTGGAC AAGAGTAGAA TCCTATGG 28
<210> 4
   <211> 27
   <212> DNA
   <213> Künstliche Sequenz
<220>
   <223> Primer
<400> 4
   TTCTCCAAGG AGCTGCAGCC ACTCTGG 27
<210> 5
   <211> 37
   <212> DNA
   <213> Künstliche Sequenz
<220>
   <223> Primer
<400> 5
   TTTGCTCAGT GGGCAAAGGA AAGGAATATA CAATTGG 37
<210> 6
   <211> 36
   <212> DNA
   <213> Künstliche Sequenz
<220>
   <223> Primer
<400> 6
   GTTGACACTG ATTTATATGG CACAATAATT CCTCTC 36